# EUROPEAN PATENT SPECIFICATION

(11) **EP 0 914 342 B1**
(45) Date of publication and mention of the grant of the patent: **21.06.2006**
(21) Application number: 97933655.9
(22) Date of filing: 08.07.1997
(51) Int. Cl.: C07K 14/655, A61K 38/31, A23K 1/165

(54) **POLYMERIC SOMATOSTATIN DERIVATIVES USEFUL FOR PROMOTING BODY GROWTH**
POLYMERE SOMATOSTATINDERIVATE GEEIGNET ZUR FÖRDERUNG DES KÖRPERWACHSTUMS
DERIVES DE SOMATOSTATINES POLYMERIQUES UTILES POUR STIMULER LA CROISSANCE CORPORELLE

(30) Priority: 08.07.1996 IT MI961408
(43) Date of publication of application: 12.05.1999
(73) Proprietor: DOX-AL ITALIA S.p.A., I-20050 Correzzana Milano (IT)
(72) Inventor: VOLPATO, Ivo, I-06070 S. Mariano (IT); BIZZINI, Bernard, F-46100 Figeac (FR); GRABITZ, Ernst, Bernhard, I-22064 Campofiorenzo Fraz. di Casatenov (IT)
(74) Representative: Gervasi, Gemma
(86) International application number: PCT/EP1997/003605
(87) International publication number: WO 1998/001474

(56) References cited:
- DE-A- 2 718 718
- US-A- 3 845 204
- US-A- 3 863 008
- US-A- 4 803 261
- RORSTAD OP : "Evidence for the Presence of Dihydro (Reduced) Somatostatin-14 in the Guinea Pig Brain" PEPTIDES, vol. 8, 1987, pages 849-854, XP008024119
- FRANCO-BOURLAND RE & RODRIGUEZ-HERNANDEZ AO: "Effect of cysteamine on the biosynthesis and degradation of rat hypothalamic l-[355]Cys-Vasopressin, -Oxytocin, and -Somatostatin, measured after S-Carboxymethylation" SOCIETY FOR NEUROSCIENCE ABSTRACTS, vol. 12, no. 2, 9 - 14 November 1986, page 762 XP008024120

## Description

### Field of the invention

The present invention relates to polymeric somatostatin derivatives, whose administration to man and animals can significantly increase the body growth rate and index, without substantially producing important side effects.

Furthermore, the administration of L-amino acids, in particular L-arginine, preferably as arginine D,L-pyroglutamate, or of oligopeptides, in particular containing L-amino acids, (such as L-arginine) and at least a D, L, or D,L-pyroglutamic or D, L or D,L-aspartic acid residue, in combination with said chemically modified somatostatins, can produce a synergic effect on the body growth.

The pharmacological interaction with the growth indeces is highly important both in the human field, for the treatment of pathologies associated with delayed or reduced development, and in the veterinary field, for enhancing, e.g., the growth cycles of livestock.

Body growth is mainly regulated by the growth hormone (GH), synthesized in specific acidophilic cells, called somatotropic cells. constituting pituicytes to a large extent.

GH secretion is regulated by the CNS and mediated by two hypothalamic factors: the growth hormone-releasing hormone (GHRH) factor and the somatotropin release inhibiting factor (SRIF).

The metabolic and growth promotion effects deriving from GH hormone are connected with its ability to stimulate the production of somatomedins which mediate somatotropic hormone activity (W.H. Daughaday, Clin. Endocrinol. Metabl., (1977), 6, 117).

Several compounds, out of which neurotransmitters, hormones and metabolites, can alter hormone secretion by acting on the hypothalamus and affecting the synthesis of GHRH or SRIF.

Furthermore, physical exercise, stress, emotional excitement, protein-rich meals are normal stimuli capable of increasing hormone secretion.

### Prior art

Several pharmacological approaches meant for stimulating body growth through mechanisms involving the secretion and activation of endogenous somatotropic hormone are already known. A. treatment consists in the administration of the growth hormone itself, extracted from human matter, reproduced *in vitro* from cultured pituitary cells, or obtained by the recombinant DNA technique.

The recombination derivatives known so far, defined as synthetic, are methionyl-GH (Somatrem ® ), which is much more antigenic than its natural analogue, obtained by extraction, and a recombination analogue having a lower immunogenic power, denominated somatotropin (Somatotropin®).

GH and the derivatives thereof are absorbed by the intramuscular or subcutaneous route (see Goodman & Gilman's. The Pharmacological Basis of Therapeutics, 8th Ed., 1990, p. 1341). However, hypothyroidism and, frequently, antibodies production may follow the treatment with GH (D.R. Marshk. D.T. Liu, eds., Banbury Report 29: Therapeutic Peptides and Proteins: Assessing the New Technologies, Cold Spring Harbor Lab., N.Y., 1988).

Another treatment consists in the immunization to the growth hormone itself that, by feedback, may increase the endogenous somatotropic activity (D. Flint, AFRC News, 4th July, 1989).

The active immunization of animals with peptides corresponding to GH epitopes may cause the production of antibodies capable of increasing the somatotropic activity of endogenous GH (A. Holder, J. Ivany, R. Aston, Molec. Immunol., (1987), 24, 143).

The growth hormone may also be controlled by a direct action on the modulators of its biosynthesis.

Structural analogues of the growth hormone-releasing hormone (GHRH), composed of a chain of 29 amino acids and exhibiting the same activity as the natural hormone, have been recently synthesized.

The administration of GHRH synthetic analogue to man by the intravenous route rapidly stimulates the growth hormone release. However, in a long-term treatment, GH secretion is pulsatile with simultaneous occurrence of inhibitory effects.

Somatostatin (Somatotropin Release Inhibiting Factor - SRIF) is produced by the pancreatic islands as a response to the many stimuli and hormones capable of provoking insulin and GH secretion. The hormone also inhibits several gastrointestinal secretions, e.g. of gastrin, gastric juice, secretin, CCK, vasoactive intestinal peptide (VIP) and digestive enzymes; possible consequences may be malabsorption and further side effects.

Somatostatin is biosynthesized as a precursor of 116 amino acids, which are split by proteolysis into peptides consisting of 14 or 28 amino acid residues, the active form being that having 14 amino acids. The following schemes report the structure of somatostatin consisting of 14 amino acid residues, or somatostatin-14, available e.g.. under the trademark Sigma ® 9129 (cf. Biochemicals Catalogue, ICN, 1992-1993. p. 1009): 
S——————————————————————S
|                                                                    |
Ala-Gly-Cys-Lys-Asn-Phe-Phe-Trp-Lys-Thr-Phe-Thr-Ser-Cys
1    2    3    4    5    6    7    8    9    10   11   12   13   14
 and the structure of somatostatin consisting of 28 amino acids, also known as prosomatostatin or somatostatin-28 (cf. Biolchemical Catalogue, ICN. 1992-1993, p. 1009):

Ser-Ala-Asn-Ser-Asn-Pro-Ala-Met-Ala-Pro-Arg-Glu-Arg-Lys-
1 2 3 4 5 6 7 8 9 10 11 12 13 14

S                                                    S
-Ala-Gly-Cys-Lys-Asn-Phe-Phe-Trp-Lys-Thr-Phe-Thr-Ser-Cys
15   16   17   18   19   20   21   22   23   24   25   26   27   28

Biologically active, physiological somatostatins, of synthetic or extractive origin, are already known.

Somatostatin produces pharmacological effects by linking to specific surface receptors of target cells.

The influence of arginine on the glutamine brain levels and its action as an antagonist to depression-causing substances are known. Said effects are significantly strengthened when the amino acid is administered as a salt with D,L-pyroglutamic acid (P.M. Provenzano, A. Brucato. S. Gianguzza, A. Coppola, G. Orzalesi. R. Selleri, F. Innocenti, and I. Volpato, Arzneim. Forsch./Drug Res., (1977). 27(II), 8, p. 1533).

Aspartate is found in very high concentrations in brain. It has excitatory effects on neurons.

The existence of selective and high affinity aspartate uptake systems support its role as excitatory neurotransmitter (Goodman & Gilman's, 8th Ed., pag. 257-259).

USP 3,926,937 describes polymeric reduced somatostatin containing 2-100 repeating units, obtained by oxidizing reduced (linear) somatostatin to produce disulfide linkages with the free mercapto groups of the cysteine moieties at position 3 and 14. This prior art somatostatin derivative inhibits the release of growth hormone in mammals, and is useful in the treatment of acromegaly, excessive statural growth, and vascular complication of diabetes, e.g. blindness.

USP 3,912,807 relates to the use of insulin in combination with reduced (linear) somatostatin or polymeric reduced somatostatin, such as that according to USP 3,926,937 in the treatment of diabetes mellitus.

### Technical Problem

The inconveniences that may be brought about by the aforementioned treatments may be recapitulated as follows:
a. great risks are involved in the pharmacological correction of GH lack, promoted by a substitutive treatment: i.e., the production of antibodies and a series of cascade reactions with consequent inhibition of the endogenous biosynthesis and simultaneous activation of the biosynthesis of inhibitors;
b. similar mechanisms are generated by administering the main modulators, activators and inhibitors of the biosynthesis of the hormone itself, GHRH and SRIF;
c. at the present state, a pharmacological aid derived from nutritional factors is very complex, since similar families of nutrients may simultaneously influence the biosynthesis of the hormone and of its main inhibitor.

### Summary

It has surprisingly been found that polymeric somatostatin derivatives , characterized in that they are the product of polymerization of somatostalin 1-14 of Structural formula I :can promote body growth in man and animals and that the administration of said chemically modified somatostatins in combination with L-amino acids in the L-form or in any mixture with their D-form, such as the racemic D,L form, in particular L- or D,L-arginine, or L- or D,L-aspartic acid, or with oligopeptides produces a synergic effect. 
Ala Gly Cys Lys Asn Phe Phe Trp Lys Thr Phe Thr Ser Cys
S S
 or of somatostatin 1-28 of structure formula II: 
Ser Ala Asn Ser Asn Pro Ala Met Ala Pro Arg Glu Arg Lys Ala
Gly Cys Lys Asn Phe Phe Trp Lys Thr Phe Thr Ser Cys
S S
 in the presence of a dialdehyde.

The present invention relates to polymerization product of somatostatin having 14 or 28 amino acids in the presence of a dialdehyde.

It is a further object of the present invention to provide a method of preparation of the aforesaid somatostatins, the pharmaceutical compositions containing the same and their use in all human and animal pathological and non-pathological situations, wherein body growth and development is to be promoted, in particular in the preparation of a composition for treatment of pathologies associated with a delayed or reduced body development of man and animals; in the veterinary field, in the situations wherein livestock growth cycles are to be accelerated.

The present invention also includes the use of the claimed derivatives of somatostatins combined with at least a compound selected from the group consisting of L-amino acids and any mixture thereof with their D-enantiomers, such as their racemic D,L mixtures, oligopeptides other than somatostatin derivatives containing at least a D, L or D,L-pyroglutamic acid residue or at least a D, L or D,L aspartic acid residue and at least an L-amino acid residue, oligopeptides other than somatostatins containing at least a basic L-amino acid residue, and the salts thereof with pharmaceutically acceptable acids or bases, in all situations as defined above.

Further objects of the present invention may be inferred from the present text.

### Detailed description of the invention

For the sake of simplicity, the somatostatins consisting of 14 or 28 amino acids are designated somatostatins-14 or -28, respectively.

Somatostatins useful as starting materials for the preparation of the present somatostatin derivatives modified by treatment with chemical reagent are biologically active somatostatins, i.e. exhibiting the physiological activity typical of endogenous somatostatin, and can be obtained e.g. by synthesis, by extraction of animal tissue (e.g. mammal tissues, such as bovine or porcine extractive somatostatins), or by recombinant DNA tecnique.

The polymeric somatostatin derivatives of the present invention are typically obtained by treating the somatostatins in the presence of dialdehyde.

Extractive somatostatins useful as starting materials for the present invention are for instance obtained as described by B.D. Noe. J. Spless. J.E. Rivier, and W. Vale, Endocrinol., (1979), 105 , p. 1410; in any case, commercially available somatostatins can be used as starting materials.

According to a typical embodiment of the present invention, polymerized somatostatin derivatives are, e.g., prepared by treating an endogenous, biologically active somatostatin-14 or -28, preferably having 14 amino acids, with a dialdehyde, such as glutaraldehyde. The method adopted may be, e.g., as described by S. Avrameas, Immunochemistry, Pergamon Press (1969), 6, 43-52.

For example, somatostatin (e.g. extractive) is treated, in an aqueous medium, with glutaraldehyde, at a pH generally ranging from 6.0 to 8.0. preferably at a pH of 6.7-6.9 (e.g. in a phosphate buffer), at a temperature ranging from 4°C to 40°C, typically at room temperature (+20°C/+25°C). The reaction is complete in about 0.5 to 5 h, typically in 3 h.

Glutaraldehyde is typically used in a stoichiometric excess with respect to the amino-NH₂ groups of somatostatin (typically 10⁻⁴ to 10⁻⁵ mol glutaraldeyde per 10⁻⁵/10⁻⁶ moles of -NH₂ groups of somatostatin).

According to a particular embodiment of the present invention, treatment with glutaraldehyde is followed by treatment of the reaction mixture with tyrosine, preferably added in essentially the same molar excess as that of glutaraldehyde with respect to somatostatin. At the reaction end, glutaraldehyde excess is neutralized by treatment with lysine, e.g. under the same reaction conditions adopted for the polymerization.

The resulting polymerization product is preferably purified by dialysis, typically on membranes with a molecular exclusion limit (cutoff) of about 500 to 1,000 Daltons, preferably versus a buffer having a pH of 6.0 to 8.0, e.g., versus a buffer having a pH of 7.0 to 7.5, such as PBS (physiological solution/phosphate buffer). The dialysis, which is preferably carried out at a cool temperature, e.g. of 4°C to 10°C. allows the purification of the final product from glutaraldehyde.

The resulting product exhibits a degree of polymerization preferably ranging from about 3 to 8 repeating units.

The polymeric somatostatin derivatives of the present invention find application, in particular:
a. in the human field, to correct growth defects;
b. in zootechny, to accelerate livestock growth curves and to increae body weight.

In the treated subjects, administration of the claimed derivatives stimulates body growth without producing evident side effects.

No alteration to the quality of livestock meat takes place.

The derivatives of somatostatins according to the present invention are typically administered by the parenteral way, in particular by the intramuscular (i.m.) or subcutaneous way (s.c.).

The claimed pharmaceutical compositions are prepared by conventional techniques, as reported, e.g., in Remington's Pharmaceutical Sciences, 18th Ed., 1990. They include at least a derivative of somatostatins according to the present invention, in a therapeutically effective quantity, in combination with one or more pharmaceutically acceptable excipients or diluents, or mixtures thereof.

Particularly preferred are the preparations for injection, comprising at least a somatostatin derivative of the invention and at least a diluent, wherein said derivative is dispersed, dissolved or emulsified, e.g. water for injection, and optionally other excipients.

The present preparations for injection can be, e.g., in the form of vials for the simultaneous preparation of the liquid to be injected. e.g. comprising a first vial enclosing the somatostatin derivative in the solid form and a second vial enclosing the diluent to be added to the first vial immediately prior to administration.

Sustained release preparations for injection are also preferred, which can be for instance prepared by methods analogous to those known for the preparation of the slow-releasing forms of insulin.

The Applicants have surprisingly found that the administration of the aforementioned L-amino acids optionally admixed with their D enantiomers, e.g. as D,L mixtures, and oligopeptides, in combination with the present derivatives of somatostatins, can synergically strengthen their effect. Furthermore, the administration of same in the salified form with pyroglutamic acid (herein abbreviated as Pyr) or with aspartic acid, promotes growth to a significantly larger extent than the administration of amino acids as free bases.

Pyroglutamic acid and aspartic acid can be in the D or L forms or mixtures thereof; typically they are in the racemic form (D,L), abbreviated as D,L-Pyr and D, L-Asp.

Preferably, the L-amino acids useful as growth promoters are the natural ones, and are preferably basic (having other basic groups in addition to the α-NH₂ group), such as L-arginine and L-lysine. Out of the amino acids to be combined with the present somatostatin derivatives, L-arginine is preferred, and the L-arginine salts with D,L-pyroglutamic acid or with D,L-aspartic acid are particularly preferred; D,L-pyroglutamic acid was known as a neurotrophic agent, and D.L-aspartic acid as neurotransmitter, but their use as adjuvants to body growth stimulation was neither described nor suggested.

Oligopeptides are preferably dipeptides, tripeptides and tetrapeptides, and contain natural L-amino acids. They preferably contain at least a residue selected out of L-arginine, L-lysine and mixtures thereof.

The oligopeptides containing at least a basic amino acid contain in particular one or more residues of L-arginine, L-lysine or mixtures thereof. An example is Lys-Arg-Arg.

To the Applicants' knowledge, the oligopeptides containing at least a pyroglutamic acid residue are novel. They are obtained by conventional methods. Said oligopeptides typically contain only one D, L or D,L pyroglutamic acid residue or one D, L or D, L aspartic acid residue, situated at the beginning of the amino acidic sequence. Preferred examples are:
D,L-Pyr-Arg-Arg
D.L-Pyr-Arg-Arg-Arg
D.L-Asp-Arg.

The aforesaid L-amino acids, oligopeptides and salts thereof are preferably administered daily by the oral route, either as such or as pharmaceutical compositions, or, preferably, are directly added to the diet as dietary compositions or supplements, especially in the zootechnical field. The dietary supplement can be for instance a concentrated supplement (e.g. a premix) for mixing or blending with animal feed. The dietary composition may be e.g. feedstuff added with the aforesaid L-aminoacids and oligopeptides.

The pharmaceutical compositions containing the aforesaid L-amino acids, oligodipeptides or salts thereof, combined with one or more pharmaceutically acceptable excipients, prepared according to conventional techniques, constitute a further object of the present invention.

The aforesaid L-amino acids and oligopeptides may also be administered by injection; in this case, they are preferably administered after administration of the present derivatives of somatostatins, generally 6-8 hours after or 24 hours after somatostatin derivatives administration.

Typically, the present derivatives of somatostatins are administered by the parenteral route, in combination with the aforesaid amino acids, oligopeptides, or salts thereof, which are administered by the oral route.

Typical dosages of the derivatives of somatostatins of the invention and of the aforesaid L-amino acids and oligopeptides or salts thereof, as defined above, are reported below:
A. Somatostatin derivatives:
   a. Use in the human field, by the parenteral route (typically subcutaneous or intramuscular):
      the daily dosage rates correspond, e.g., to the doses of somatostatin administered for treating gigantism;
      the daily dose is preferably partitioned into one or more doses and repeated .every 7-15 days.
   b. Use in the veterinary field, by the parenteral (typically s.c.) way:
      5 0.001 to 2 mg per kg/bodyweight per day, as a single or repeated dose, depending on the animal species, typically once for poultry and rabbits, and every 15-30 days for swine, ovines and cattle, and every 7-15 days in man.

      The administration of a slow-releasing pharmaceutical form (e.g. analogous to the slow-releasing forms of insulin) is preferred.
B. L-amino acids and salts thereof, oligopeptides as defined above and salts thereof:
   in the human and veterinary fields:
   5 to 50 mg/kg/bodyweight per day by the oral route (or 1 to 10 mg/kg/bodyweight per day by the i.m. route).
C. Treatment duration: it varies depending on the species; it averagely lasts from 15 days to 3 months according to the results. In man, the treatment may be repeated in cycles, e.g., in yearly cycles.

### Experimental Part

### Example 1 - Preparation of D,L-pyroglutamic acid salt with L-arginine

L-arginine was dissolved in little boiling water and added, still in the hot, with a concentrated aqueous solution of D,L-pyroglutamic acid (1:1 molar ratio). The mixture was left in the hot under stirring for some minutes, concentrated on rotary evaporator at a reduced pressure until total water removal. The residue was taken up three times with abs. ethanol, filtered and evaporated again, each time by the aforesaid method. Final yield was 84% of the theoretical value.

### Example 2 - Preparation of a somatostatin derivative polymerized in the presence of glutaraldehyde (inactivated somatostatin)

Reagents : Extractive somatostatin-14 (Sigma R 9129) (M.W.= 1637.9; 1 mg corresponds to 6.1 x 10⁻⁷ mole).

Glutaraldehyde (M.W. = 100; 1 mg corresponds to; 1 x 10⁻⁵ mole)

Tyr (M.W. = 181; 1 mg corresponds to 5.52 x 10⁻⁶ mole).

Lys (M.W. = 146; 1 mg corresponds to 6.85 x 10⁻⁶ mole).

Process: A mixture of 10 mg of somatostatin-14 (6.1 x 10⁻⁶ mole) + 5 ml H₂O + 5 ml PBS is added dropwise with glutaraldehyde (50 mg; 200 µl of a 25% solution; 0.61 x 10⁻³ mole, corresponding to a 1:50 excess with respect to Lys).

A precipitate forms. The reaction mixture is left under stirring for about 5 minutes at the laboratory temperature. Then, it is added with Tyr (100 µl of a 100 mg/ml Tyr solution), and left under stirring for 10 minutes at room temperature.

The reaction is then blocked by Lys addition (680 µl of a 2M lys solution). pH (6.25) was then adjusted to 6.95 by diluted NaOH addition. The reaction mixture is filtered on a 0.45 µm filter, stored at < 4°C and used as such.

### Efficacy Treatment Control

Six groups of rats during their growth stage (12 animals each group) were treated as follows.

### Experimental model:

- group I : placebo animals; normal feeding conditions;
- group II : animals treated subcutaneously only with a single dose of polymerized somatostatin (PS) according to Example 2;
- group III : animals daily treated only with PGA-Arg (L-arginine D,L-pyroglutamate, with a 1:1 arginine: pyroglutamic acid molar ratio), by means of a gastric probe;
- group IV : animals daily treated s.c. with a single dose of polymerized somatostatin (PS) according to Example 2, and also with PGA-Arg, per os, daily, for the duration of the treatment;
- group V : animals treated s.c. with a single dose of polymerized somatostatin (PS) according to Example 2, and also with ASP-Arg (L-arginine D,L-aspartate, with a 1:1 arginine: aspartic acid molar ratio), per os, daily, for the duration of the treatment;
- group VI : animals treated s.c. with two doses of polymerized somatostatin (PS) according to Example 2 (the first on day 0 and the second on day 10 from the beginning of the treatment), and also with PGA-Arg, by means of a gastric probe, daily, for the duration of the treatment.

### Treatment duration : 21 days.

The single dose of polymerized somatostatin was administered at day 0, and aminoacids were administered 6-8 hours after polymerized somatostatin (PS).

### Administered dosages:

- Polymerized somatostatin (PS) : 0.005 mg/animal;
- PGA-Arg : 1 mg/animal/day per os;
- ASP-Arg : 1 mg/animal/day per os.

### Tested parameters:

- weight variation;
- feedstuff intake.

Results The results are hereinbelow reported in Tables 1 and 2.

Table 1 reports the body weight values at day 0, 7, 14 and 21.

**TABLE 1**

| Group | Treatment | Body weight (g) ¹ | | | |
|---|---|---|---|---|---|
| | | Day → 0 | 7 | 14 | 21 |
| I | Placebo | 92 | 140 | 177 | 225 |
| II | PS s.c. | 93 | 140 | 195 | 259 |
| III | PGA-Arg | 92 | 140 | 183 | 238 |
| IV | PS+PGA-Arg | 92 | 140 | 198 | 275 |
| V | PS+ASP-Arg | 93 | 140 | 205 | 280 |
| VI | 2xPS+PGA-Arg | 93 | 140 | 200 | 285 |

| | | | | | |
|---|---|---|---|---|---|
| 1 Averages values Eₓ/n | | | | | |
| PS = Polymerized Somatostatin | | | | | |
| PGA-Arg = arginine pyroglutamate | | | | | |
| ASP-Arg = arginine aspartate | | | | | |

Table 2 reports the percent body weight increase with respect to the 0 day weight. The amounts in brackets refer to the difference of percent weight increase with respect to placebo group I.

**TABLE 2**

| Group | Treatment | Percent body weight increase | | | | |
|---|---|---|---|---|---|---|
| | | Day -> 7 | 14 | | 21 | |
| I | Placebo | 52.1 | 92.4 | | 144.6 | |
| II | PS s.c. | 50.5 | 109.6 | (17.2) | 178.5 | (33.5) |
| III | PGA-Arg | 52.1 | 98.9 | (6.5) | 158.6 | (14.0) |
| IV | PS+PGA-Arg | 52.1 | 115.2 | (22.8) | 198.9 | (54.3) |
| V | PS+ASP-Arg | 50.5 | 120.4 | (28.0) | 201.1 | (56.5) |
| VI | 2xPS+PGA-Arg | 50.5 | 115.1 | (22.7) | 206.5 | (61.8) |

| | | | | | | |
|---|---|---|---|---|---|---|
| Note : The daily feed intake is superimposable for all groups. Comments : Polymerized somatostatin (PS) is able to significantly increase growth with respect to control animals. | | | | | | |

## Claims

1. Polymeric somatostatin derivative, **characterized in that** it is the product of polymerization of somatostatin 1-14 of structural formula I: 
Ala Gly Cys Lys Asn Phe Phe Trp Lys Thr Phe Thr Ser Cys
S S
 or of somatostatin 1-28 of structural fonnula II: 
Ser Ala Asn Ser Asn Pro Ala Met Ala Pro Arg Glu Arg Lys Ala
Gly Cys Lys Asn Phe Phe Trp Lys Thr Phe Thr Ser Cys
S S
 in the presence of a dialdehyde.

2. Polymeric somatostatin derivative according to claim 1, wherein the dialdehyde is glutaraldehyde.

3. Polymeric somatostatin derivative according to claim 1, **characterized in that** it has a degree of polymerization ranging from 3 to 8 repeating units.

4. A process for the preparation of a polymeric somatostatin derivative according to claim 1.

5. The process according to claim 4, wherein the dialdehyde is glutaraldehyde.

6. The process according to claim 5, wherein somatostatin is treated with glutaraldehyde in aqueous medium, at a pH ranging from 6.0 to 8.0, at a temperature ranging from 4°C to 40°C.

7. The process according to claim 6, wherein pH is 6.7-6.9 and the temperature is room temperature (20°C to 25°C).

8. The process according to any of claim 4-7, wherein glutaraldehyde is used in a stoechiometric excess with respect to the -NH₂ groups of somatostatin.

9. The process according to any of claim 4-8, wherein glutaraldehyde is used in quantity of from 10⁻⁴ to 10⁻⁵ moles glutaraldehyde per 10⁻⁵/10⁻⁶ moles -NH₂ groups of somatostatin.

10. The process according to claim 4, which further comprises treating the reaction mixture with tyrosine, after treatment with glutaraldehyde.

11. The process according to claim 10, wherein tyrosine is added in the same molar excess as that of glutaraldehyde.

12. The process according to claim 4, which further comprises neutralizing glutaraldehyde excess by treatment with lysine at the end of polymerization.

13. The process according to claim 4, which further comprises purifying the polymeric somatostatin derivative by dialysis on membranes with molecular exclusion limit ("cut-off") of 500 to 1000 Daltons, versus a buffer having pH of from 7.0 to 7.5.

14. The process according to claim 13, wherein dialysis is carried out at a temperature of from 4°C to 10°C.

15. Polymeric somatostatin derivative, obtainable according to the process defined in any of claims from 5 to 14

16. A pharmaceutical or dietary composition comprising at least a polymeric somatostatin derivative as defined in any of claims 1-3 or 15, and at least one pharmaceutically acceptable excipient, diluent or mixture thereof.

17. The pharmaceutical composition according to claim 16, which is a preparation for injection.

18. The pharmaceutical composition according to claim 16. which is a sustained release preparation.

19. Use of at least one polymeric somatostatin derivative as defined in any of claims 1-3 or 15, for the preparation of a pharmaceutical or dietary composition for the treatment of pathological or non-pathological situations wherein body growth and development is to be promoted.

20. The use according to claim 19, wherein said situations are pathologies associated with delayed or reduced body development in man and animals.

21. The use according to claim 19, wherein said situations are those wherein livestock growth cycles are to be accelerated.

22. The use according to claim 19, in combination with a compound selected among i) L-amino acid; ii)oligopeptides other than somatostatin and derivatives thereof containing at least one D or L or D,L aspartic acid residue and at least one L-amino acid residue; iii) oligopeptides other than somatostatin and derivatives thereof containing at least one basic L-amino acid residue; iv) salt of species (i), (ii) or (iii) with pharmaceutically acceptable acids or bases.

23. The use according to claim 22, wherein the compound is L-arginine or L-lysine.

24. The use according to claim 22, wherein the compound is L-arginine, salified with D,L-pyroglutamic acid or D,L-aspartic acid.

25. The use according to claim 22,wherein the oligopeptide is selected among dipeptides, peptides and tetrapeptides.

26. The use according to claim 22, wherein the oligopeptide comprises at least one residue selected among L-arginine, L-lysine and mixtures thereof.

27. The use according to claim 22, wherein the oligopeptide comprises only one D or L or D,L pyroglutamic acid or D,L-aspartic acid residue, situated at the beginning of the amino acid.

28. The use according to claim 22 wherein the oligopeptide is selected among: D,L-Pyr-Arg-Arg, D,L-Pyr Arg-Arg-Arg, Lys-Arg Arg, and D,L-Asp-Arg, wherein D,L-Pyr is D,L-pyrogiLdamic acid and D,L-Asp is D,L-aspartic acid.

29. Pharmaceutical or dietary composition according to claim 16, further comprising a compound selected among I) L-amino acid; ii)oligopeptide other than somatostatin and derivatives thereof containing at least one D or L or D,L pyroglutamic acid residue, or at least one D or L or D,L aspartic acid residue and at least one L-amino acid residue; ii) oligopeptide other then somatostatin and derivatives thereof containing at least one basic L-amino acid residue; iv) salt of species i), ii) or iii) with pharmaceutically acceptable acids or bases.

30. A kit comprising of a product according to claim 16, and in addition to a compound selected among i) L-amino acid; ii)oligopeptide other than somatostatin and derivatives thereof containing at least one D or L or D,L pyroglutamic acid residue, or at least one D or L or D,L aspartic acid residue and at least one L-amino acid residue; ii) oligopeptide other than somatostatin and derivatives thereof containing at least one basic L-amino acid residue; iv) salt of species i), ii) or iii) with pharmaceutically acceptable acids or bases

## Patentansprüche

1. Polymeres Somatostatinderivat, **dadurch gekennzeichnet, dass** es sich um das Produkt der Polymerisation von Somatostatin 1-14 mit Strukturformel I: 
Ala Gly Cys Lys Asn Phe Phe Trp Lys Thr Phe Thr Ser Cys
S S
 oder Somatostatin 1-28 mit Strukturformel II: 
Ser Ala Asn Ser Asn Pro Ala Met Ala Pro Arg Glu Arg Lys Ala
Gly Cys Lys Asn Phe Phe Trp Lys Thr Phe Thr Ser Cys
S S
 in Gegenwart von Dialdehyd handelt.

2. Polymeres Somatostatinderivat gemäß Anspruch 1, wobei es sich bei dem Dialdehyd um Glutaraldehyd handelt.

3. Polymeres Somatostatinderivat gemäß Anspruch 1, **dadurch gekennzeichnet, dass** es einen Polymerisationsgrad im Bereich von 3 bis 8 in sich wiederholenden Einheiten aufweist.

4. Verfahren für die Herstellung von polymerem Somatostatinderivat gemäß Anspruch 1.

5. Verfahren gemäß Anspruch 4, wobei es sich bei dem Dialdehyd um Glutaraldehyd handelt.

6. Verfahren gemäß Anspruch 5, wobei Somatostatin mit Glutaraldehyd in wässriger Lösung in einem pH-Bereich von 6,0 bis 8,0 und einem Temperaturbereich von 4°C bis 40°C behandelt wird.

7. Verfahren gemäß Anspruch 6, wobei der pH-Wert im Bereich von 6,7 bis 6,9 und die Temperatur im Bereich der Raumtemperatur (20°C bis 25°C) liegt.

8. Verfahren gemäß einem der Ansprüche 4-7, wobei Glutaraldehyd in stöchiometrischem Überschuss bezogen auf die -NH₂-Gruppen von Somatostatin verwendet wird.

9. Verfahren gemäß einem der Ansprüche 4-8, wobei Glutaraldehyd in einer Quantität von 10⁻⁴ bis 10⁻⁵ Mol an Glutaraldehyd pro 10⁻⁵/10⁻⁶ Mol -NH₂-Gruppen von Somatostatin verwendet wird.

10. Verfahren gemäß Anspruch 4, welches im Weiteren nach der Behandlung mit Glutaraldehyd die Behandlung der Reaktionsmischung mit Tyrosin umfasst.

11. Verfahren gemäß Anspruch 10, wobei Tyrosin im gleichen molaren Überschuss wie Glutaraldehyd zugegeben wird.

12. Verfahren gemäß Anspruch 4, welches im Weiteren die Neutralisierung des Glutaraldehydüberschusses durch Behandlung mit Lysin am Ende der Polymerisation umfasst.

13. Verfahren gemäß Anspruch 4, welches im Weiteren die Reinigung des polymeren Somatostatinderivates durch Membrandialyse mit einer molekularen Ausschlussgrenze ("Cut-Off") von 500 bis 1000 Dalton gegen einen Puffer mit einem pH-Wert im Bereich von 7,0 bis 7,5 umfasst.

14. Verfahren gemäß Anspruch 13, wobei die Dialyse bei einer Temperatur im Bereich von 4°C bis 10°C durchgeführt wird.

15. Polymeres Somatostatinderivat, erhältlich gemäß dem Verfahren, das in einem der Ansprüche von 5 bis 14 definiert ist.

16. Pharmazeutische Zusammensetzung oder Nahrungsmittelzusammensetzung umfassend mindestens ein polymeres Somatostatinderivat wie in einem der Ansprüche 1-3 oder 15 definiert und mindestens einen pharmazeutisch akzeptablen Trägerstoff, Verdünner oder Mischung daraus.

17. Pharmazeutische Zusammensetzung gemäß Anspruch 16, wobei es sich um eine Zubereitung für die Injektion handelt.

18. Pharmazeutische Zusammensetzung gemäß Anspruch 16, wobei es sich um eine Zubereitung mit kontrollierter Freisetzung handelt.

19. Verwendung von mindestens einem polymeren Somatostatinderivat, wie definiert in einem der Ansprüche 1-3 oder 15, für die Herstellung einer pharmazeutischen Zusammensetzung oder Nahrungsmittelzusammensetzung zur Behandlung pathologischer oder nicht-pathologischer Zustände, bei denen das Körperwachstum und die Entwicklung gefördert werden sollen.

20. Verwendung gemäß Anspruch 19, wobei es sich bei den genannten Zuständen um Krankheiten handelt, die mit verzögerter oder eingeschränkter körperlicher Entwicklung bei Mensch und Tier in Verbindung stehen.

21. Verwendung gemäß Anspruch 19, wobei es sich bei den genannten Zuständen um solche handelt, bei denen die Entwicklungszyklen von Nutztieren beschleunigt werden.

22. Verwendung gemäß Anspruch 19, in Kombination mit einer Verbindung ausgewählt unter i) L-Aminosäure; ii) Oligopeptiden anders als Somatostatin und Derivate davon enthaltend mindestens einen D- oder L- oder D,L-Asparaginsäurerest und mindestens einen L-Aminosäurerest; iii) Oligopeptide anders als Somatostatin und Derivate davon enthaltend mindestens einen basischen L-Aminosäurerest, iv) Salz der Verbindungen (i), (ii) oder (iii) mit pharmazeutisch akzeptablen Säuren oder Basen.

23. Verwendung gemäß Anspruch 22, wobei es sich bei der Verbindung um L-Arginin oder L-Lysin handelt.

24. Verwendung gemäß Anspruch 22, wobei es sich bei der Verbindung um L-Arginin handelt, umgewandelt in ein Salz mit D,L-Pyroglutaminsäure oder D,L-Asparaginsäure.

25. Verwendung gemäß Anspruch 22, wobei das Oligopeptid ausgewählt wird unter Dipeptiden, Peptiden und Tetrapeptiden.

26. Verwendung gemäß Anspruch 22, wobei das Oligopeptid mindestens einen Rest ausgewählt unter L-Arginin, L-Lysin und Mischungen davon enthält.

27. Verwendung gemäß Anspruch 22, wobei das Oligopeptid nur eine D- oder L- oder D,L-Pyroglutaminsäure oder einen D,L-Asparaginsäurerest enthält, lokalisiert am Anfang der Aminosäure.

28. Verwendung gemäß Anspruch 22, wobei das Oligopeptid ausgewählt wird unter: D,L-Pyr-Arg-Arg, D,L-Pyr-Arg-Arg-Arg, Lys-Arg-Arg und D,L-Asp-Arg, wobei D,L-Pyr D,L-Pyroglutaminsäure und D,L-Asp D,L-Asparaginsäure ist.

29. Pharmazeutische Zusammensetzung oder Nahrungsmittelzusammensetzung gemäß Anspruch 16 im Weiteren umfassend eine Verbindung ausgewählt unter i) L-Aminosäure; ii) Oligopeptid anders als Somatostatin und Derivate davon, enthaltend mindestens eine D oder L oder D,L-Pyroglutaminsäurerest oder mindestens einen D- oder L- oder D,L-Asparaginsäurerest und mindestens einen L-Aminosäurerest; ii) Oligopeptid anders als Somatostatin und Derivate davon, enthaltend mindestens einen basischen L-Aminosäurerest; iv) Salz der Verbindungen i), ii) oder iii) mit pharmazeutisch akzeptablen Säuren oder Basen.

30. Ein Kit beinhaltend ein Produkt gemäß Anspruch 16, und zusätzlich zur gewählten Verbindung ausgewählt unter i) L-Aminosäure; ii) Oligopeptid anders als Somatostatin und Derivate davon, enthaltend mindestens einen D- oder L- oder D,L-Pyroglutaminsäurerest oder mindestens einen D- oder L- oder D,L-Asparaginsäurerest und mindestens einen L-Aminosäurerest; ii) Oligopeptide anders als Somatostatin und Derivate davon, enthaltend mindestens einen basischen L-Aminosäurerest; iv) Salz der Verbindungen i), ii) oder iii) mit pharmazeutisch akzeptablen Säuren oder Basen.

## Revendications

1. Dérivé polymère de somatostatine, **caractérisé en ce qu'**il est le produit de polymérisation de la somatostatine 1-14 de formule structurale 1 : 
Ala Gly Cys Lys Asn Phe Phe Trp Lys Thr Phe Thr Ser Cys
S S
 ou de la somatostatine 1-28 de formule structurale II : 
Ser Ala Asn Ser Asn Pro Ala Met Ala Pro Arg Glu Arg Lys Ala
Gly Cys Lys Asn Phe Phe Trp Lys Thr Phe Thr Ser Cys
S S
 en présence d'un dialdéhyde.

2. Dérivé polymère de somatostatine selon la revendication 1, dans lequel le dialdéhyde est le glutaraldéhyde.

3. Dérivé polymère de somatostatine selon la revendication 1, **caractérisé en ce qu'**il a un degré de polymérisation compris dans la gamme de 3 à 8 motifs répétés.

4. Procédé de préparation d'un dérivé polymère de somatostatine selon la revendication 1.

5. Procédé selon la revendication 4, dans lequel le dialdéhyde est le glutaraldéhyde.

6. Procédé selon la revendication 5, dans lequel la somatostatine est traitée avec du glutaraldéhyde en milieu aqueux à un pH compris dans la gamme de 6,0 à 8,0, à une température comprise dans la gamme de 4°C et 40°C.

7. Procédé selon la revendication 6, dans lequel le pH est de 6,7 à 6,9 et la température est la température ambiante (20°C à 25°C).

8. Procédé selon l'une quelconque des revendications 4 à 7, dans lequel le glutaraldéhyde est utilisé en un excès stoechiométrique par rapport aux groupes -NH₂ de la somatostatine.

9. Procédé selon l'une quelconque des revendications 4 à 8, dans lequel le glutaraldéhyde est utilisé en une quantité de 10⁻⁴ à 10⁻⁵ moles de glutaraldéhyde par 10⁻⁵/10⁻⁶ moles de groupes -NH₂ de la somatostatine.

10. Procédé selon la revendication 4, qui comprend de plus le traitement du mélange réactionnel avec de la tyrosine, après le traitement avec le glutaraldéhyde.

11. Procédé selon la revendication 10, dans lequel la tyrosine est ajoutée avec le même excès molaire que celui du glutaraldéhyde.

12. Procédé selon la revendication 4, qui comprend de plus la neutralisation de l'excès de glutaraldéhyde par traitement avec de la lysine à la fin de la polymérisation.

13. Procédé selon la revendication 4, qui comprend de plus la purification du dérivé polymère de somatostatine par dialyse sur des membranes ayant une limite d'exclusion moléculaire (seuil de coupure) de 500 à 1000 Dalton, par rapport à un tampon ayant un pH de 7,0 à 7,5.

14. Procédé selon la revendication 13, dans lequel la dialyse est effectuée à une température de 4°C à 10°C.

15. Dérivé polymère de somatostatine pouvant être obtenu par le procédé défini dans l'une quelconque des revendications 5 à 14.

16. Composition pharmaceutique ou diététique comprenant au moins un dérivé polymère de somatostatine tel que défini dans l'une quelconque des revendications 1 à 3 ou 15, et au moins un excipient, un diluant ou un mélange de ceux-ci acceptables du point de vue pharmaceutique.

17. Composition pharmaceutique selon la revendication 16, qui est une préparation pour injection.

18. Composition pharmaceutique selon la revendication 16, qui est une préparation à libération prolongée.

19. Utilisation d'au moins un dérivé polymère de somatostatine tel que défini dans l'une quelconque des revendications 1 à 3 ou 15, pour la préparation d'une composition pharmaceutique ou diététique destinée au traitement de situations pathologiques ou non pathologiques dans lesquelles la croissance et le développement corporels doivent être activés.

20. Utilisation selon la revendication 19, dans laquelle lesdites situations sont des pathologies associées à un développement retardé ou réduit chez l'homme et les animaux.

21. Utilisation selon la revendication 19, dans laquelle lesdites situations sont celles dans lesquelles les cycles de croissance des animaux sur pied doivent être accélérés.

22. Utilisation selon la revendication 19, en association avec un composé choisi parmi : (i) un L-aminoacide ; (ii) des oligopeptides autres que la somatostatine et ses dérivés contenant au moins un résidu d'acide aspartique D ou L ou D,L et au moins un résidu de L-aminoacide ; (iii) des oligopeptides autres que la somatostatine et ses dérivés contenant au moins un résidu de L-aminoacide basique ; (iv) un sel des espèces (i), (ii) ou (iii) formé avec des acides ou des bases acceptables du point de vue pharmaceutique.

23. Utilisation selon la revendication 22, dans laquelle le composé est la L-arginine ou la L-lysine.

24. Utilisation selon la revendication 22, dans laquelle le composé est la L-arginine, salifiée avec l'acide D,L-pyroglutamique ou l'acide D,L-aspartique.

25. Utilisation selon la revendication 22, dans laquelle l'oligopeptide est choisi parmi des dipeptides, des peptides et des tétrapeptides.

26. Utilisation selon la revendication 22, dans laquelle l'oligopeptide comprend au moins un résidu choisi par la L-arginine, la L-lysine et leurs mélanges.

27. Utilisation selon la revendication 22, dans laquelle l'oligopeptide comprend seulement un résidu d'acide D ou L ou D,L-pyroglutamique ou d'acide D,L-aspartique, situé au début de l'acide aminé.

28. Utilisation selon la revendication 22, dans laquelle l'oligopeptide est choisi parmi : D,L-Pyr-Arg-Arg, D,L-Pyr-Arg-Arg-Arg, Lys-Arg-Arg et D,L-Asp-Arg, où D,L-Pyr est l'acide D,L-pyroglutamique et D,L-Asp est l'acide D,L-aspartique.

29. Composition pharmaceutique ou diététique selon la revendication 16, comprenant de plus un composé choisi parmi : (i) un L-aminoacide ; (ii) un oligopeptide autre que la somatostatine et ses dérivés contenant au moins un résidu D ou L ou D,L-pyroglutamique ou au moins un résidu D ou L ou D,L-aspartique et au moins un résidu de L-aminoacide ; (iii) un oligopeptide autre que la somatostatine et ses dérivés contenant au moins un résidu de L-aminoacide basique ; (iv) un sel des espèces (i), (ii) ou (iii) formé avec des acides ou des bases acceptables du point de vue pharmaceutique.

30. Kit comprenant un produit selon la revendication 16, et en plus un composé choisi parmi : (i) un L-aminoacide ; (ii) un oligopeptide autre que la somatostatine et ses dérivés contenant au moins un résidu D ou L ou D,L-pyroglutamique ou au moins un résidu D ou L ou D,L-aspartique et au moins un résidu de L-aminoacide ; (iii) un oligopeptide autre que la somatostatine et ses dérivés contenant au moins un résidu de L-aminoacide basique ; (iv) un sel des espèces (i), (ii) ou (iii) formé avec des acides ou des bases acceptables du point de vue pharmaceutique.
